# EUROPEAN PATENT SPECIFICATION

(11) **EP 1 720 518 B1**
(45) Date of publication and mention of the grant of the patent: **12.08.2009**
(21) Application number: 05723611.9
(22) Date of filing: 24.02.2005
(51) Int. Cl.: A61K 9/00, A61K 31/734, A61K 47/36

(54) **ALGINATE VISCOELASTIC COMPOSITION, METHOD OF USE AND PACKAGE**
VISKOELASTISCHE ALGINAT-ZUSAMMENSETZUNG, HERSTELLUNGSVERFAHREN UND VERPACKUNG
COMPOSITION VISCO-ELASTIQUE A BASE D'ALGINATE, METHODE D'UTILISATION ET EMBALLAGE

(30) Priority: 26.02.2004 US 547855 P
(43) Date of publication of application: 15.11.2006
(73) Proprietor: BAUSCH & LOMB INCORPORATED, Rochester, NY 14604-2701 (US)
(72) Inventor: JANI, Dharmendra, Fairport, NY 14450 (US); SALAMONE, Joseph, C., Boca Raton, FL 33486 (US)
(74) Representative: Maiwald, Walter
(86) International application number: PCT/US2005/005800
(87) International publication number: WO 2005/082333

(56) References cited:
- WO-A-02/40056
- WO-A-98/41171
- US-A- 4 997 652
- US-A1- 2001 016 772

## Description

### BACKGROUND OF THE INVENTION

### Field of the Invention

This invention relates to a viscoelastic composition, method of use and related device used in viscosurgical applications and more particularly to a viscoelastic composition used in ophthalmic surgical application such as cataract removal surgery.

### Discussion of Related Art

In the past decade, advances in the technology of eye surgery have made surgical treatment of eye disease and deformities attractive to alternative therapies. Cataract removal is one of the more common surgical procedures. Cataracts are opacities of the ocular lens, which generally arise in the elderly. Typically, cataract surgery involves removal of the cataractous lens from the capsular bag and replacement of the cataractous lens with a synthetic intraocular lens. Presently, this procedure involves making an incision through the sclera or cornea into the anterior chamber of the patient's eye. Another incision is made into the capsular bag. The cataractous lens is fractured in the capsular bag by procedures such as phacoemulsification and removed from the capsular bag by procedures such as aspiration. Thereafter, an intraocular lens is inserted into the capsular bag and deployed therein. The overall procedure is potentially traumatic to the tissue surrounding the anterior chamber. It is advantageous to reduce the amount of trauma to any living tissue in the patient eye during a surgical procedure. Particularly, corneal endothelial cells in the capsular bag are sensitive to damage, which is often irreversible. Serious damage can cause loss of eyesight and failure of the surgical procedure.

Viscoelastic compositions are injected in the anterior chamber of the eye and the capsular bag during surgery to protect the tissue from physical trauma. The viscoelastic compositions provide a physical barrier or cushion between the instruments and the tissue. Furthermore, viscoelastic compositions maintain the shape of a cavity during operation including the anterior chamber and capsular bag.

In addition to cataract surgery, viscoelastic compositions are useful in reducing tissue trauma and maintaining space of a cavity during other ophthalmic surgical procedures, including but not limited to trabeculectomy and vitrectomy.

Viscoelastic materials have properties that make them effective for use in eye surgery to maintain the shape of a cavity and to protect the tissue. A viscoelastic under zero-shear or low-shear preferably has a relatively high viscosity. Higher viscosity compounds under zero-shear or low-shear conditions have better space maintenance properties than low viscosity compounds. However, it is difficult to inject or remove a highly viscous liquid through a cannula used for surgical procedures inside the eye. It is highly desirable to have a compound that has low viscosity under high-shear conditions and high viscosity under zero-shear or low-shear conditions. The ratio of the shear rate at low-shear condition to a high-shear condition is the pseudoplasticity index. It is desirable for a viscoelastic material to have high pseudoplasticity.

Common viscoelastic compositions for eye surgery include sodium hyaluronate (Healon® by Pfizer, New York, New York), sodium hyaluronate and chondroitin sulfate (Viscoat® by Alcon Laboratories, Fort Worth, Texas), hydroxypropylmethylcellulose (Ocucoat® by Bausch & Lomb, Rochester, New York).

A composition whose viscoelastic component is essentially sodium hyaluronate has good shape maintaining characteristics, but is less effective at protecting the cells against damage during phacoemulsification.

A composition with hydroxypropylmethylcellulose and mixtures of hyaluronic acid and chondroitin sulfate are two viscoelastic compositions with dispersive viscoelastic properties. However, there is still a need for a dispersive viscoelastic with a relatively flat low-shear viscosity profile and improved dampening characteristics.

Alginate, for the purpose of this application is a polysaccharide that comprises β-D-mannuronic acid and α-L-guluronic acid monomers or salts or derivatives of such acids or salts.

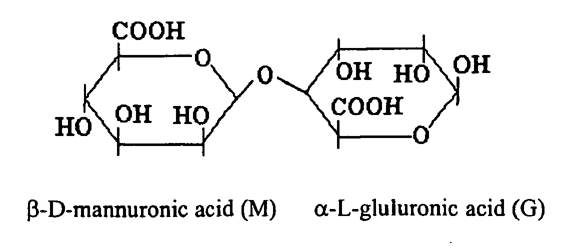

Some alginate polymers are block copolymers with blocks of the guluronic acid (or salt) monomers alternating with blocks of the mannuronic acid (or salt) monomers. Some alginate molecules have single monomers of guluronic acid (or salt) alternating with the comonomers of mannuronic acid (or salt). The ratio and distribution of the M and G components along with the average molecular weight affect the physical and chemical properties of the copolymer. See Haug, A. et al., Acta Chem Scand 20:183-190 (1966). Alginate polymers have viscoelastic rheological properties and other properties that make it suitable for some medical applications. See Klock, G. et al., Biocompatibility of manurononic acid-rich alginates, Biomaterials 18(10): 707-713 (1997).

The use of alginate as a thickener for topical ophthalmic use is disclosed in U.S. Patent No. 6,528,465 and U.S. Publication 2003-0232089 incorporated herein by reference in their entirety. In U.S. Patent No. 5,776,445, alginate is used as a drug delivery agent that is topically applied to the eye. Particularly, the amount of guluronic acid in the alginate was taught to exceed 50%.

While significant improvements have been made in the rheological properties of viscoelastic compositions, there still exists a need for a composition that has good adhesive properties improved dampening ability. The present invention addresses these and other needs.

### SUMMARY OF THE INVENTION

The present invention is a novel viscoelastic composition that has improved viscoelastic properties. The composition comprises an aqueous vehicle and a viscosurgically pure alginate with a minimum alginate concentration of about 0.01 %w/v and a maximum alginate concentration of about 20 %w/v based upon the total weight of the composition. Particularly, the viscoelastic compositions have relatively flat low-shear viscosity profile. Furthermore, the viscoelastic compositions or materials of at least one embodiment of the present invention have damping characteristics that are an improvement over dispersive viscoelastic compositions or materials for viscosurgical applications.

In another embodiment of the present invention, there is a method of temporarily maintaining space in a cavity in mammalian tissue. The method comprises injecting a viscoelastic material comprising alginate and an aqueous carrier into the cavity. At least a portion of the viscoelastic material is removed from the cavity. The alginate is viscosurgically pure.

In yet another embodiment, there is a method of protecting tissue from trauma during a surgical procedure, the method comprises coating at least a portion of the tissue with a viscoelastic material comprising an aqueous vehicle and alginate. After the tissue is coated, a surgical procedure is performed near the tissue. At least a portion of the viscoelastic material is removed from the tissue after surgical procedure is performed. In one embodiment, at least a portion of the tissue in an anterior chamber of an eye is covered during the coating step. In another application, at least a portion of the corneal endothelium of an eye is coated.

In still another embodiment, there is a package for a viscoelastic material. The package comprises a syringe containing a viscoelastic material comprising an aqueous vehicle and alginate. Optionally, the syringe has an outlet port. The package further comprises a cannula configured to sealably connect to the outlet port having a maximum inner diameter of about 1000 microns. Typically, the maximum inner diameter is about 700 microns, about 500 microns or about 300 microns.

In one embodiment, there is a method of replacing a natural lens from an eye, the method comprises the steps of:
(a) providing a passage through a sclera or cornea into an anterior chamber of the eye;
(b) removing at least a portion of the aqueous humor from the anterior chamber;
(c) inserting a viscoelastic material into the anterior chamber, the viscoelastic material comprises an aqueous vehicle and alginate;
(d) phacoemulsifying a lens in the capsular bag of the eye;
(e) removing substantially all of the lens from the capsular bag;
(f) injecting the viscoelastic material into the capsular bag; and
(g) inserting an intraocular lens into the capsular bag.

In one embodiment, there is an additional step of removing at least a portion of the viscoelastic material from the capsular bag after the intraocular lens is inserted into the capsular bag. Optionally and additionally, at least a portion of the viscoelastic material is removed from the anterior chamber. The phrase, "removing substantially all" as it relates to lenses and lens fragments, means a sufficient quantity to facilitate an effective removal of the lens. According to one embodiment, an effective removal of the lens requires removal of a minimum of 90%w/v of the lens, 95%w/v of the lens or 98 %w/v of the lens. Typically, the method further includes a step of suturing the sclera after the intraocular lens is inserted into the capsular bag.
In one embodiment, there is a method of inserting an intraocular lens into a capsular bag of an eye. The method comprises the steps of:
providing an eye with a cornea removed from the capsular bag and a passage through the sclera or cornea into the capsular bag;
providing a lens insertion device comprising a loadable chamber configured to receive the intraocular lens, a tapered conduit having a first end connected to the loadable chamber and a second end, the second end is configured to penetrate into the passage, and a slidable actuator configured to actuate the intraocular lens through the conduit past the second end;
coating at least a portion of the intraocular lens with a viscoelastic material comprising an aqueous vehicle and alginate;
loading the intraocular lens into the loadable chamber;
inserting the conduit into the passage;
positioning the second end inside the capsular bag;
actuating the coated intraocular lens through the conduit into the capsular bag; and
removing the conduit from the passage.

In one application, the step of coating occurs after the step of loading. Additionally and optionally, the second end of the tapered conduit has an inner diameter that is a maximum of about 5 mm. Preferably the second end of the tapered conduit has an inner diameter that is a maximum of about 4 mm about 3.5 mm, about 3 mm or about 2.8 mm.

### BRIEF DESCRIPTION OF THE DRAWINGS

Figure 1 is a graph showing steady state shear of one alginate solution.
Figure 2 is a graph showing oscillation test results for one alginate solution.

### DETAILED DESCRIPTION OF THE INVENTION

The present invention is a novel viscoelastic composition that has improved viscoelastic properties. The composition comprises an aqueous vehicle and a viscosurgically pure alginate with a minimum alginate concentration of about 0.01 %w/v and a maximum alginate concentration of about 20 %w/v based upon the total weight of the composition. Particularly, the viscoelastic compositions or materials of at least one embodiment of the present invention is capable of maintaining a zero-shear viscosity profile at higher shear rates relative to other leading viscoelastic materials. Furthermore, the viscoelastic compositions or materials of at least one embodiment of the present invention have a damping ratio that is higher than other viscoelastic compositions or materials for viscosurgical applications.

For the purpose of this application, a viscoelastic material has relatively viscous properties under low-shear and relatively elastic properties under high-shear conditions.

Viscosurgically pure as it pertains to this application refers to a viscoelastic composition or ingredient thereof that is sufficiently pure and free of impurities to meet or exceed the United States Food and Drug Administration standards for a viscosurgical viscoelastic effective at the time this application is effective.

Alginate is a polysaccharide that comprises β-D-mannuronic acid and α-L-guluronic acid monomers or salts or derivatives of such acids or salts. Some alginate polymers are block copolymers with blocks of the guluronic acid (or salt) monomers alternating with blocks of the mannuronic acid (or salt) monomers. Some alginate molecules have single monomers of guluronic acid (or salt) alternating with the comonomers of mannuronic acid (or salt). Other variations and combinations of mannuronic acid and guluronic acid are also potentially found in the alginate polymers. Alginate is typically extracted from sources of brown seaweed including kelp. Sources for alginate include but are not limited to alginate under the trademark Pronova UP™ from FMC Biopolymers, FMC Corporation, Philadelphia, Pennsylvania.
The viscoelastic composition according to one embodiment of the present invention includes an alginate that comprises β-D-mannuronic acid and α-L-guluronic acid. Particularly, the ratio of the β-D-mannuronic acid to α-L-guluronic acid is in a range having a minimum of about 1 and a maximum of about 4. Typically, the ratio of β-D-mannuronic acid to α-L-guluronic acid is in a range having a minimum of about 1.1 or about 1.2. Typically, the ratio of β-D-mannuronic acid to α-L-guluronic acid is in a range having a maximum of about 3.4, about 3, about 2 or about 1.3. Most preferably, in one embodiment, the ratio of β-D-mannuronic acid to α-L-guluronic acid is in a range having a minimum of about 1.2 and a maximum of about 1.3. Other variations and combinations of mannuronic acid and guluronic acid are also potentially found in synthetic or natural sources of alginate.

The average molecular weight of the alginate is a minimum of about 50kD and a maximum of about 5,000kD. Typically, the average molecular weight of the alginate is a minimum of about 50kD, about 100kD, about 200kD, about 500kD or about 1000kD. Typically, the average molecular weight of the alginate is a maximum of about 2000kD, about 1000kD, about 750kD or about 500kD.

As noted the viscoelastic composition has a minimum alginate concentration of about 0.05 %w/v and a maximum alginate concentration of about 9 %w/v, based upon the total weight of the composition. Typically, the minimum alginate concentration is about 1 %w/v, about 1.5 %w/v, about 2 %w/v, about 3 %w/v or about 4 %w/v based upon the total of the viscoelastic composition or material. Typically, the maximum alginate concentration is about 10 %w/v, about 8 %w/v, about 6 %w/v, about 4 %w/v, about 3 %w/v or about 2 %w/v based upon the total weight of the viscoelastic composition or material. Preferably the alginate concentration is a minimum of about 2%w/v and a maximum of about 5.25%w/v.

Preferably, in one embodiment, the average molecular weight of the alginate is a minimum of about 295kD and a maximum of about 350kD when the alginate concentration is a minimum of about 1%w/v and a maximum of about 3%w/v and most preferably about 2%w/v. Preferably, in one embodiment, the average molecular weight of the alginate is a minimum of about 200kD to a maximum of about 300kD when the alginate concentration is about 4%w/v to about 6%w/v, most preferably about 5%w/v.

Optionally, the pH is adjusted to a desired range having a minimum of about 7 and a maximum of about 8. In one embodiment, the pH of the viscoelastic composition or material is a minimum of about 7.1, about 7.2 or about 7.3 and a maximum of about 7.8, about 7.6, about 7.4 or about 7.3. The pH is adjusted with physiological acids or bases such as acetic acid, acetate, carbonic acid, carbonate, phosphoric acid, phosphate. After the pH is adjusted, the pH is typically maintained with a buffer system. Preferably, a buffer system does not substantially affect the viscoelastic properties of the viscoelastic composition or material. Desirably, the buffer system does not cause irritation at the amounts used in the viscoelastic composition or material. Buffer systems useful in the present invention include but are not limited to a N-2hydroxyethylpiperazine-N'-ethane sulphonic acid (HEPES) buffer system, a carbonate buffer system, and a phosphate buffer system--more preferably a phosphate buffered saline (PBS) system.

In one embodiment, the osmolality of the composition is a minimum of about 200mOsmol/L and a maximum of about 400mOsmol/L. Typically, the osmolality of the viscoelastic composition or material is a minimum of about 220mOsmol/L, about 260mOsmol/L, about 280mOsmol/L, about 300mOsmol/L or about 320mOsmol/L. Typically, the osmolality of the viscoelastic composition or material is a maximum of about 400mOsmol/L, about 380mOsmol/L, about 360mOsmol/L or about 340mOsmol/L. Most preferably, the osmolality of the viscoelastic composition is about 340mOsmol/L. In one embodiment, the osmolality is altered by adding an osmolality-adjusting agent known in the art. Typically, osmolality-adjusting agents are capable of increasing the osmolality of the viscoelastic composition or material without causing irritation of the eye at the quantity needed to appropriately adjust the osmolality. Suitable osmolality-adjusting agents include but are not limited to glycerin. Most preferably, the osmolality-adjusting agent is added in an amount that is a minimum of about 0.1%w/v, about 1%w/v or about 1.5%w/v and a maximum of about 5%w/v, about 2.5%w/v or about 2%w/v.

The viscoelastic properties of the viscoelastic composition of the present invention are important to their effectiveness in the surgical procedure. Zero-shear viscosity is a good indicator of how a viscoelastic material will maintain the space of a cavity in human tissue. Zero-shear viscosity is the extrapolation of the viscosity of a liquid to a zero-shear rate from measurements of viscosity as the shear rate approaches zero measured on a plate and cone rheometer at 37°C. In one embodiment, the zero-shear viscosity of the composition is a minimum of about 10Pa-s and a maximum of about 300Pa-s. Generally, the zero-shear viscosity of the viscoelastic composition or material is a minimum of about 50Pa-s, about 75Pa-s or about 100Pa-s. Generally, the zero-shear viscosity of the viscoelastic composition or material is a maximum of about 250Pa-s, about 200Pa-s or about 150Pa-s.

High-shear conditions refer to shear conditions having a minimum shear force of about 100sec⁻¹. High-shear viscosity, for the purpose this patent application, is the viscosity of a liquid measured on a plate and cone rheometer at 37°C with a shear rate of 1000sec⁻¹. According to one embodiment, the high-shear viscosity of the composition is a minimum of about 0.1Pa-s and a maximum of about 30Pa-s. Generally, the high-shear viscosity of the viscoelastic composition or material is a minimum of about 0.5Pa-s, about 1Pa-s or about 2Pa-s. Generally, the high-shear viscosity of the viscoelastic composition or material is a maximum of about 20Pa-s, about 15Pa-s, about 10Pa-s, about 5Pa-s or about 3Pa-s.

The pseudoplasticity index is another important factor. The pseudoplasticity measures the degree of change in viscosity from a low shear state to a high shear state. For the purpose of this application, pseudoplasticity is defined as the ratio of viscosity at a shear rate of 0.3s⁻¹ to the viscosity at a shear rate of 300s⁻¹. According to one embodiment, the pseudoplasticity index of the viscoelastic composition is a minimum of about 80. Typically, the pseudoplasticity index of the viscoelastic composition is a minimum of about 100, about 120, about 140, about 160 or about 180. In one embodiment, the pseudoplasticity index of the viscoelastic composition is about 200.

In one embodiment, there is a method of replacing a natural lens from an eye. Examples of procedures for removing a lens from a patient's eye include but are not limited to U.S. Patent Nos. 3,589,363 (cataract surgery), 3,693,613 (phacoemulsification) and 5,718,676 (process using micro flow needle), which are all incorporated herein by reference in their entirety. The process generally includes providing a passage through a sclera or cornea into an anterior chamber of the eye. The process involves making a small incision into the sclera or cornea. Alternatively or additionally, a cannula or trochar is used to create a passage through the sclera or cornea. Preferably, the incision or passage is as small as possible. Preferably the incision or passage is smaller than about 5 mm, about 4 mm or about 3mm. Thereafter, the aqueous humor is withdrawn or otherwise removed from the anterior chamber of the eye.

A viscoelastic material, according to any one of the embodiments or combinations, is inserted into the anterior chamber. The viscoelastic, of one embodiment, maintains the space in the anterior chamber. The viscoelastic of one embodiment, coats the tissue in the wall of the anterior chamber.

According to one embodiment, there is a device for delivering a viscoelastic composition or material into the anterior chamber of a patient's eye. Alternatively, there is a package for viscoelastic material. The package or device comprises a syringe containing a viscoelastic material comprising an aqueous vehicle and alginate. In one embodiment, the syringe has an outlet port, the package further comprising a cannula configured to sealably connect to the outlet port having a maximum inner diameter of about 1000 microns. Typically, the maximum inner diameter is about 700 microns, about 500 microns or about 300 microns.

Once the viscoelastic material is inserted into the anterior chamber the corneal lens is removed. The technique for removing the lens includes performing a capsulorhexis incision and breaking down the lens into smaller pieces through phacoemulsification or other known techniques. Thereafter, the pieces are removed by aspiration.

The viscoelastic material is inserted into the capsular bag for space maintenance purposes. Moreover, the viscoelastic composition or material coats the capsular bag and protects it for additional steps in the surgical procedure.

According to one embodiment, the intraocular lens is inserted into the capsular bag. Typically, there is a method of inserting an intraocular lens into a capsular bag of an eye. The method comprises providing a lens insertion device comprising a loadable chamber configured to receive the intraocular lens, a tapered conduit having a first end connected to the loadable chamber and a second end. The second end is configured to penetrate through the passage in the corneal lens and into the capsular bag. An example of a lens insertion device is found in U.S. Patent No. 6,558,419, which is incorporated herein by reference in its entirety. The lens insertion device is further configured with a slidable actuator. The slidable actuator of one embodiment is configured to actuate the intraocular lens through the conduit past the second end. Typically, the second end of the tapered conduit has an inner diameter that is a maximum of about 5 mm. Preferably the second end of the tapered conduit has an inner diameter that is a maximum of about 4 mm about 3.5 mm, about 3 mm or about 2.8 mm.

Prior to deployment, at least a portion of the intraocular lens is coated with a viscoelastic composition or material according to any one of the embodiments of the present invention. The intraocular lens is loaded into the loadable chamber either before or after it is coated. The conduit is inserted through the passage. The actuator forces the intraocular lens through the passage and into the capsular bag. After the intraocular lens is deployed, the conduit is removed from the passage.

Typically, at least a portion of the viscoelastic material is removed from the capsular bag and/or anterior chamber. A physiological solution is then used to fill the anterior chamber. The sclera and/or cornea are sutured to close the passage.

In one embodiment, of the present invention, one or more viscoelastic compositions set forth in the present invention are used to maintain the space of a cavity in a patient's tissue. The process includes injecting a viscoelastic material comprising alginate and an aqueous carrier into the cavity. After the cavity is maintained for a period of time, at least a portion of the viscoelastic material is removed from the cavity. The space is often maintained during a surgical procedure that often occurs in the cavity itself. In one embodiment, the surgery occurs in the patient's eye. In another embodiment, the surgical procedure is cataract removal. The cavity is the anterior chamber of the patient's eye and/or the capsular bag of the patient's eye.

The use of alginate in surgery also protects tissue from damage during the surgical procedure. The viscoelastic composition or material coats the surface of the tissue. A surgical procedure is performed near the tissue. The viscoelastic composition cushions the tissue from physical trauma. Preferably, the viscoelastic has dispersive viscoelastic properties to protect the tissue. In one embodiment, the process of coating covers at least a portion of the tissue in an anterior chamber of an eye. In another embodiment, the step of coating covers at least a portion of the corneal endothelium of an eye. The surgical procedure further includes removing at least a portion of the viscoelastic material from the tissue.

### Example 1

Several viscoelastic polysaccharides were compared to determine rheological properties.

The following solutions were prepared for testing:
Solution 1: 2% Cross-linked Carboxymethylcellulose (Akucell from Akzo Nobel) with PBS buffer at pH 7.3.
Solution 2: 2% Hydroxypropylmethylcellulose with PBS buffer at pH 7.3.
Solution 3: 3% Alginate solution (satialgine, obtained from Degussa Texturants) with PBS buffer at 7.3.
Solution 4: Sodium Hyaluronate (Amvisc™ Plus, from Bausch & Lomb).
Solution 5: 3% Sodium Hyaluronate and 4% Chondroitin Sulfate (Viscoat^{®} Alcon Labs).

### Steady Shear Test

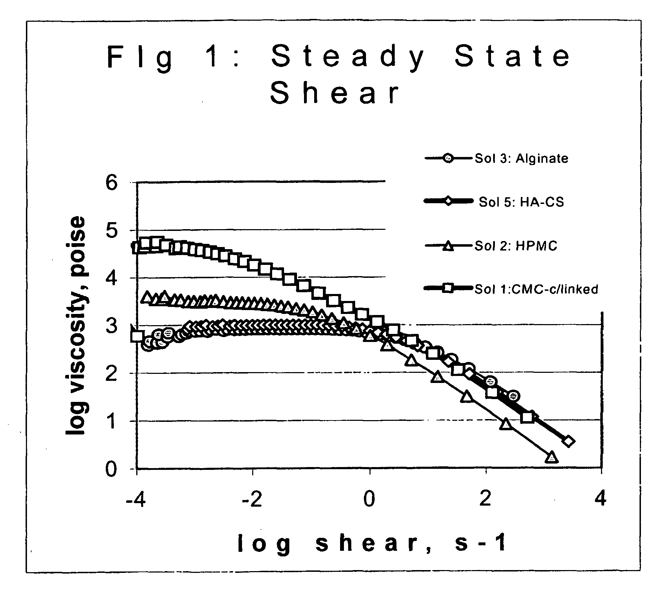

A TA Instruments T-1000R rheometer with a 50-mm diameter cone-and-plate geometry (cone angle: 2°) was used to perform rheological tests on the above solutions under ambient conditions. The geometry gap used was 48 microns. Steady shear tests were conducted using torque as the control parameter. The results of the steady shear test are shown in Fig. 1. The steady shear test illustrated that the flat zero-shear viscosity profile, similar to Solution 5 (hyaluronic acid and chondroitin sulfate), establishes that Solution 3 (alginate) has good dispersive viscoelastic. Other viscoelastic solutions tested such as Solution 1 (carboxymethylcellulose), Solution 2 (hyroxypropylmethylcellulose), Solution 4 (hyaluronic acid) does not exhibit the flat low-shear viscosity profile necessary for a dispersive viscoelastic to protect the endothelial layer during the phacoemulsification process.

### Example 3: Dynamic Oscillation Test

Dynamic oscillation tests were carried out under all the conditions of Example 2, and were additionally carried out at 1 % strain control for Solution 2, Solution 3 and Solution 5.

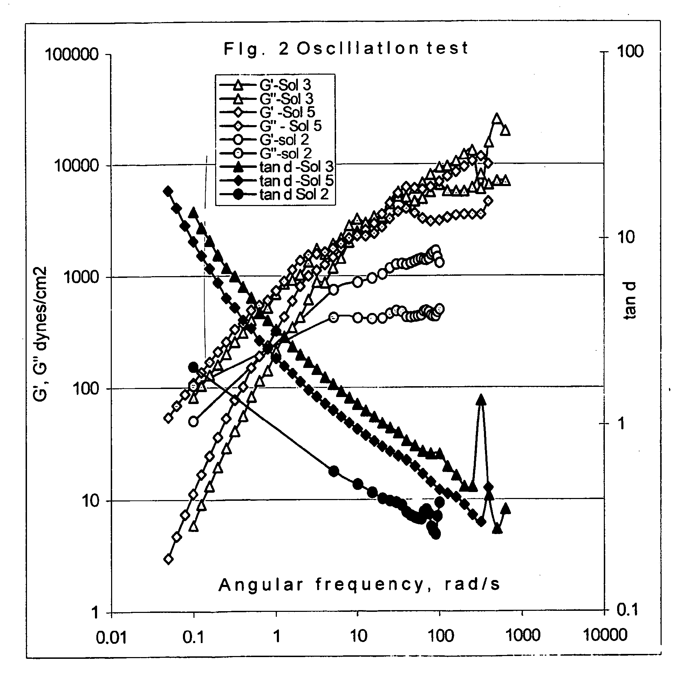

The results of the dynamic oscillation test are shown in Fig. 2, which compares the dynamic storage and loss modulus as a function of angular frequency. Solution 3 (alginate) demonstrated a similar storage and loss modulus profile as compared to Solution 5 (hyaluronic acid-chondroitin sulfate) for most of the frequency sweep. The crossover frequency for G' and G" are similar for the two viscoelastics. A comparison of the damping ratio, tanδ (= G"/G') profiles indicates a better damping or input stress dissipation ability of Solution 3 relative to Solution 5. Of particular interest is the damping region around the peak at 315 rad/s for Solution 3, which indicates that Solution 3 will dissipate the phaco-energy more effectively than Solution 5, especially at high ultrasonic frequencies, which will in turn prevent damage to the endothelial cells.

## Claims

1. A composition comprising an aqueous vehicle and a viscosurgically pure alginate, wherein the alginate comprises β-D- mannuronic acid and α-L-guluronic acid, wherein the ratio of β-D-mannuronic acid to α-L-guluronic acid is from 1 to 4, and a minimum alginate concentration of 0.05 % w/v and a maximum alginate concentration of 9 % w/v based upon the total weight of the composition, wherein the viscoelastic composition has a mininimum pseudoplasticity index of about 100.

2. The composition of claim 1, wherein the average molecular weight of the alginate is a minimum of 50kD and a maximum of 5,000 kD.

3. The composition of claim 1, wherein the osmolality of the composition is a minimum of 200 mOsmol/L and a maximum of 400 mOsmol/L.

4. The composition of claim 1, wherein the zero-shear viscosity of the composition is a minimum of 10 Pa·s and a maximum of 300 Pa·s.

5. The composition of claim 1, wherein the high-shear viscosity of the composition, measured on a plate and cone rheometer at 37°C with a shear rate of 1000 sec⁻¹, is a minimum of 0.1 Pa·s and a maximum of 30 Pa·s.

6. The composition of claim 1, wherein the pH of the composition is a minimum of 7 and a maximum of 8.

7. A composition according to claim 1 for use in a method of temporarily maintaining space in a cavity in mammalian tissue, the method comprising the steps of :
(a) injecting a said composition into the cavity; and
(b) removing at least a portion of the composition from the cavity.

8. The composition of claim 7, wherein the average molecular weight of the alginate is a minimum of 50kD and a maximum of 5,000 kD.

9. The composition of claim 7, wherein the zero-shear viscosity of the composition is a minimum of 10 Pa·s and a maximum of 300 Pa·s.

10. The composition of claim 7, wherein the high-shear viscosity of the composition, measured on a plate and cone rheometer at 37°C with a shear rate of 1000 sec⁻¹, is a minimum of 0.1 Pa·s and a maximum of 30 Pa·s.

11. A composition according to claim 1 for use in a method of protecting tissue from trauma during a surgical procedure, the method comprising the steps of:
(a) coating at least a portion of the tissue with said composition;
(b) performing a surgical procedure near the tissue after the step (a) coating; and
(c) removing at least a portion of the composition from the tissue before the step (b) performing.

12. The composition of claim 11, wherein the step (a) coating covers at least a portion of the tissue in an anterior chamber of an eye.

13. A package for a viscoelastic material, the package comprising a syringe containing a composition as claimed in claims 1 to 6.

14. The package of claim 13, wherein the syringe has an outlet port, the package further comprising a cannula configured to sealably connect to the outlet port having a maximum inner diameter of about 1000 microns.

15. A composition comprising an aqueous vehicle and a viscosurgically pure alginate with a minimum alginate concentration of 1 %w/v and a maximum alginate concentration of 3 %w/v, wherein the viscoelastic composition has a minimum pseudoplasticity index of about 100, a zero-shear viscosity of from 10 Pa·s to 300 Pa·s, and a high-shear viscosity of from 0.1 Pa·s to 30 Pa·s.

16. The composition of claim 15, wherein the zero-shear viscosity of the viscoelastic material is a minimum of 50 Pa·s and a maximum of 200 Pa·s.

17. The composition of claim 15, wherein the high-shear viscosity of the viscoelastic material, measured on a plate and cone rheometer at 37 °C with a shear rate of 1000 sec⁻¹, is a minimum of 1 Pa·s and a maximum of 20 Pa·s.

18. The composition of claim 15, wherein the alginate comprises units of β-D-mannuronic acid and α-L-guluronic acid, wherein the unit ratio of β-D-mannuronic acid to α-L-guluronic acid is from 1 to 3.

19. The composition of claim 1, wherein the viscosurgically pure alginate concentration is from 1.0 %w/v to 3 %w/v, and the average molecular weight is from 295 kD to 350 kD.

20. The composition of claim 18, wherein the average molecular weight is from 295 kD to 350 kD.

## Patentansprüche

1. Eine Zusammensetzung beinhaltend einen wässrigen Trägerstoff und ein viscochirurgisch reines Alginat, wobei das Alginat β-D-Mannuronsäure und α-L-Guluronsäure beinhaltet, wobei das Verhältnis von β-D-Mannuronsäure zu α-L-Guluronsäure von 1 bis 4 reicht und eine minimale Alginatkonzentration von 0,05 % Gewicht pro Volumen und eine maximale Alginatkonzentration von 9 % Gewicht pro Volumen basierend auf dem Gesamtgewicht der Zusammensetzung aufweist, wobei die viscoelastische Zusammensetzung einen minimalen Strukturviskositätsmessindex von ungefähr 100 hat.

2. Die Zusammensetzung nach Anspruch 1, wobei das durchschnittliche Molekulargewicht des Alginats minimal 50 kD und maximal 5.000 kD ist.

3. Die Zusammensetzung nach Anspruch 1, wobei die Osmolalität der Zusammensetzung minimal 200 mOsmol/L und maximal 400 mOsmol/L ist.

4. Die Zusammensetzung nach Anspruch 1, wobei die scherungsfreie Viskosität der Zusammensetzung minimal 10 Pa·s und maximal 300 Pa·s ist.

5. Die Zusammensetzung nach Anspruch 1, wobei die Viskosität der Zusammensetzung bei hoher Scherung, gemessen mit einem Kegel-Platte-Rheometer bei 37 °C mit einer Schergeschwindigkeit von 1000 sek⁻¹, minimal 0,1 Pa·s und maximal 30 Pa·s ist.

6. Die Zusammensetzung nach Anspruch 1, wobei der pH der Zusammensetzung minimal 7 und maximal 8 ist.

7. Eine Zusammensetzung nach Anspuch 1 für den Einsatz in einer Methode zum temporären Aufrechterhalten eines Zwischenraums in einer Kavität im Säugetiergewebe, die Methode beinhaltend die Schritte von:
(a) Injektion der genannten Zusammensetzung in die Höhle und
(b) Entfernen von zumindest einem Teil der Zusammensetzung aus der Höhle.

8. Die Zusammensetzung nach Anspruch 7, wobei das durchschnittliche Molekulargewicht des Alginats minimal 50 kD und maximal 5.000 kD ist.

9. Die Zusammensetzung nach Anspruch 7, wobei die scherungsfreie Viskosität der Zusammensetzung minimal 10 Pa·s und maximal 300 Pa·s ist.

10. Die Zusammensetzung nach Anspruch 7, wobei die Viskosität der Zusammensetzung bei hoher Scherung, gemessen mit einem Kegel-Platte-Rheometer bei 37 °C mit einer Schergeschwindigkeit von 1000 sec⁻¹, minimal 0,1 Pa·s und maximal 30 Pa·s ist.

11. Eine Zusammensetzung nach Anspruch 1 für den Einsatz in einer Methode zum Schutz von Gewebe vor einem Trauma während einem chirurgischen Verfahren, die Methode bestehend aus den Schritten:
(a) Beschichten von zumindest einem Teil des Gewebes mit genannter Zusammensetzung;
(b) Durchführen eines chirurgischen Verfahrens nahe dem Gewebe nach der Beschichtung in Schritt (a) und
(c) Entfernen von zumindest einem Teil der Zusammensetzung vom Gewebe bevor Schritt (b) durchgeführt wird.

12. Die Zusammensetzung nach Anspruch 11, wobei die Beschichtung in Schritt (a) zumindest ein Teil des Gewebes in einer vorderen Augenkammer bedeckt.

13. Eine Verpackung für ein viscoelastisches Material, die Verpackung beinhaltend eine Spritze beinhaltend eine Zusammensetzung wie beansprucht in den Ansprüchen 1 bis 6.

14. Die Verpackung von Anspruch 13, wobei die Spritze eine Auslassöffnung hat, die Verpackung ferner enthaltend eine Kanüle derart aufgebaut um mit einer Auslassöffnung, die einen maximalen Innendurchmesser von ungefähr 1000 Mikrometer aufweist, versiegelbar zu verbinden.

15. Eine Zusammensetzung beinhaltend einen wässrigen Trägerstoff und ein viscochirurgisch reines Alginat mit einer minimalen Alginatkonzentration von 1 % Gewicht pro Volumen und einer maximalen Alginatkonzentration von 3 % Gewicht pro Volumen, wobei die viscoelastische Zusammensetzung eine minimalen Strukturviskositätsmesszahl von ungefähr 100, einen scherungsfreie Viskosität von 10 Pa·s bis 300 Pa·s und eine Viskosität bei hoher Scherung von 0,1 Pa·s bis 30 Pa·s hat.

16. Die Zusammensetzung nach Anspruch 15, wobei die scherungsfreie Viskosität des viscoelastischen Materials minimal 50 Pa·s und maximal 200 Pa·s ist.

17. Die Zusammensetzung nach Anspruch 15, wobei die Viskosität des viscoelastischen Materials bei hoher Scherung, gemessen mit einem Kegel-Platte-Rheometer bei 37 °C mit einer Schergeschwindigkeit von 1000 sek⁻¹, minimal 1 Pa·s und maximal 20 Pa·s ist.

18. Die Zusammensetzung nach Anspruch 15, wobei das Alginat Anteile von β-D-Mannuronsäure und α-L-Guluronsäure enthält, wobei das Verhältnis der Anteile von β-D-Mannuronsäure zu α-L-Guluronsäure von 1 bis 3 reicht.

19. Die Zusammensetzung nach Anspruch 1, wobei die Konzentration des viscochirurgisch reinen Alginats von 1 % Gewicht pro Volumen bis 3 % Gewicht pro Volumen reicht und die durchschnittliche Molekularmasse von 295 kD bis 350 kD reicht.

20. Die Zusammensetzung nach Anspruch 18, wobei die durchschnittliche Molekularmasse von 295 kD bis 350 kD reicht.

## Revendications

1. Composition comprenant un véhicule aqueux et un alginate pur d'un point de vue viscochirurgical, où l'alginate comprend de l'acide β-D-mannuronique et de l'acide α-L-guluronique, où le rapport de l'acide β-D-mannuronique sur l'acide α-L-guluronique est compris entre 1 et 4, et une concentration en alginate minimale de 0,05 % p/v et une concentration en alginate maximale de 9 % p/v en se basant sur le poids total de la composition, où la composition viscoélastique présente un indice de pseudoplasticité minimal d'environ 100.

2. Composition selon la revendication 1, où le poids moléculaire moyen de l'alginate est au minimum de 50 kD et au maximum de 5 000 kD.

3. Composition selon la revendication 1, où l'osmolarité de la composition est au minimum de 200 mOsmol/l et au maximum de 400 mOsmol/1.

4. Composition selon la revendication 1, où la viscosité à taux de cisaillement nul de la composition est au minimum de 10 Pa·s et au maximum de 300 Pa·s.

5. Composition selon la revendication 1, où la viscosité à haut taux de cisaillement de la composition, mesurée sur un rhéomètre cône-plan à 37°C avec un taux de cisaillement de 1000 sec⁻¹, est au minimum de 0,1 Pa·s et au maximum de 30 Pa·s.

6. Composition selon la revendication 1, où le pH de la composition est au minimum de 7 et au maximum de 8.

7. Composition selon la revendication 1, destinée à être utilisée dans un procédé de maintien temporaire d'un espace dans une cavité dans un tissu mammalien, le procédé comprenant les étapes consistant à :
(a) injecter ladite composition dans la cavité ; et
(b) retirer au moins une partie de la composition de la cavité.

8. Composition selon la revendication 7, où le poids moléculaire moyen de l'alginate est au minimum de 50 kD et au maximum de 5 000 kD.

9. Composition selon la revendication 7, où la viscosité à taux de cisaillement nul de la composition est au minimum de 10 Pa·s et au maximum de 300 Pa·s.

10. Composition selon la revendication 7, où la viscosité à haut taux de cisaillement de la composition, mesurée sur un rhéomètre cône-plan à 37°C avec un taux de cisaillement de 1000 sec⁻¹, est au minimum de 0,1 Pa·s et au maximum de 30 Pa·s.

11. Composition selon la revendication 1, destinée à être utilisée dans un procédé pour protéger un tissu d'un traumatisme au cours d'une procédure chirurgicale, le procédé comprenant les étapes consistant à :
(a) appliquer un revêtement sur au moins une partie du tissu avec ladite composition ;
(b) réaliser une procédure chirurgicale à proximité du tissu après l'application du revêtement de l'étape (a) ; et
(c) retirer du tissu au moins une partie de la composition avant de réaliser l'étape (b).

12. Composition selon la revendication 11, où l'application du revêtement de l'étape (a) recouvre au moins une partie du tissu dans une chambre antérieure d'un oeil.

13. Conditionnement pour un matériau viscoélastique, le conditionnement comprenant une seringue contenant une composition selon les revendications 1 à 6.

14. Conditionnement selon la revendication 13, où la seringue possède un orifice de sortie, le conditionnement comprenant en outre une canule configurée pour se raccorder, de façon hermétique, à l'orifice de sortie ayant un diamètre interne maximal d'environ 1 000 microns.

15. Composition comprenant un véhicule aqueux et un alginate pur d'un point de vue viscochirurgical, avec une concentration en alginate minimale de 1 % p/v et une concentration en alginate maximale de 3 % p/v, où la composition viscoélastique présente un indice de pseudoplasticité minimal d'environ 100, une viscosité à taux de cisaillement nul comprise entre 10 Pa·s et 300 Pa·s, et une viscosité à haut taux de cisaillement comprise entre 0,1 Pa·s et 30 Pa·s.

16. Composition selon la revendication 15, où la viscosité à taux de cisaillement nul du matériau viscoélastique est au minimum de 50 Pa·s et au maximum de 200 Pa·s.

17. Composition selon la revendication 15, où la viscosité à haut taux de cisaillement du matériau viscoélastique, mesurée sur un rhéomètre cône-plan à 37°C avec un taux de cisaillement de 1000 sec⁻¹, est au minimum de 1 Pa·s et au maximum de 20 Pa·s.

18. Composition selon la revendication 15, où l'alginate comprend des unités d'acide β-D-mannuronique et d'acide α-L-guluronique, où le rapport en unités de l'acide β-D-mannuronique sur l'acide α-L-guluronique est compris entre 1 et 3.

19. Composition selon la revendication 1, où la concentration en alginate pur d'un point de vue viscochirurgical est comprise entre 1,0 % p/v et 3 % p/v, et le poids moléculaire moyen est compris entre 295 kD et 350 kD.

20. Composition selon la revendication 18, où le poids moléculaire moyen est compris entre 295 kD et 350 kD.
